# EUROPEAN PATENT APPLICATION

(11) **EP 3 530 668 A1**
(43) Date of publication of application: **28.08.2019**
(21) Application number: 18158177.8
(22) Date of filing: 22.02.2018
(51) Int. Cl.: C07K 14/005, G01N 33/569, C07K 16/10

(54) **A NOVEL ASSAY FOR THE DIAGNOSIS OF VIRAL INFECTIONS**

(71) Applicant: Euroimmun Medizinische Labordiagnostika AG, 23560 Lübeck (DE)
(72) Inventor: The designation of the inventor has not yet been filed

(57) **Abstract**

The present invention relates to a recombinant or chemically synthesized polypeptide comprising SEQ ID NO1 or a variant thereof, a diagnostically useful carrier comprising in means for specifically capturing an antibody to SEQ ID NO1 in a sample from a subject, a kit comprising the polypeptide or a diagnostically useful carrier, and a method for diagnosing, prognosing or monitoring the treatment of a virus, preferably Flavivirus, more preferably POWV infection comprising the step detecting in the sample from a subject the presence or absence of an antibody to SEQ ID NO1 and/or an antibody to SEQ ID NO2, and a use of a polypeptide comprising SEQ ID NO1 and/or a SEQ ID NO2 or a variant thereof for the diagnosis and a use of an IgM antibody to SEQ ID NO1 or in means for specifically capturing an IgM class antibody to SEQ ID NO1 for increasing the diagnostic reliability of an immunoassay for the diagnosis of a virus infection.

## Description

The present invention relates to a recombinant or chemically synthesized polypeptide comprising SEQ ID NO1 or a variant thereof, a diagnostically useful carrier comprising in means for specifically capturing an antibody to SEQ ID NO1 in a sample from a subject, a kit comprising the polypeptide or a diagnostically useful carrier, and a method for diagnosing, prognosing or monitoring the treatment of a virus, preferably Flavivirus, more preferably POWV infection comprising the step detecting in the sample from a subject the presence or absence of an antibody to SEQ ID NO1 and/or an antibody to SEQ ID NO2, and a use of a polypeptide comprising SEQ ID NO1 and/or a SEQ ID NO2 or a variant thereof for the diagnosis and a use of an IgM antibody to SEQ ID NO1 or in means for specifically capturing an IgM class antibody to SEQ ID NO1 for increasing the diagnostic reliability of an immunoassay for the diagnosis of a virus infection.

Tick-borne diseases are a major public health concern for the great Great Lakes region of the mid-Eastern United States to the sub-Saharan deserts of Africa. Ticks carry agents cause a wide array of clinical pathologies which are protozoan bacterial or viral in origin. Among them is the Powassan virus (POWV), which was first reported in 1958 in Powassan, Ontario, US. POWV is endemic in the north-east and upper mid-west of the United States, and cases have also been reported in far-eastern Russia. Like other tick-borne diseases, POWV is a diagnostic challenge because of its versatility of clinical presentation and the unpredictability of the cause of the illness.

The symptoms of POWV infection vary from person to person, as some are asymptomatic and others have a more progressive cause, meaning that a significant number, possibly the majority of patients remain undiagnosed. However, a POWV infection not recognized as such can develop to a rapidly progressing, neurological disease. Symptoms include headache, fever, focal neurological deficits, confusion, generalized weakness, ataxia, somnolence, speech problems, aphasia and dysarthria. The fatality rate is 10%, and about 50% of those people who developed neurological symptoms end up with long-term deficits.

Among the entirety of Flaviviruses, POWV, according to phylogenetic analyses based on the sequences of glycoprotein E (GpE) and non-structural protein 1 (NS1), is one of the more distant relatives of the ZIKV (Wong, S.S., Poon, R.W., Wong, S.C. (2016) Zika virus infection-the next wave after dengue?, Journal of the Formosan Medical Association, http://doi.org/10.1016/j.jfma.2016.02.002).

Moreover, it has been assigned to the group of tick-borne Flaviviruses rather than to the group of mosquito-borne Flaviviruses including ZIKV (Mackenzie, J., Williams, D.T. (2009) The Zoonotic Flaviviruses of Southern, South-Eastern and Eastern Asia, and Australasia: The Potential for Emergent Viruses, Zoonoses and Public Health, 56(6-7), 338-56).

Unfortunately, it appears to share with the ZIKV the capacity for transplacental transmission, as well as subsequent infection and injury to the developing fetus (Platt, D.J., Smith, A.M., Arora, N., Diamond, M.S., Coyne, C.B., Miner, J.J. (2018) Zika virus-related neurotropic Flaviviruses infect human placental explants and cause fetal demise in mice, Science Translational Medicine, 10 (426)).

The diagnosis of tick-borne diseases such as POWV infection represents a clinical challenge. Imaging methods including computer tomography (CT) scans and magnetic resonance imaging (MRI) of the brain may be employed. However, acute presentations of POWV will show no abnormalities on a CT scan unless there is intraparenchymal beating and subdural haematoma. MRI cannot be employed in an emergency setting as it requires a patient to be still throughout the scan. No imaging method at present may be used to provide the specific diagnosis of an infectious disease.

According to several reports, no test for the specific diagnosis of a POWV infection based on the detection of specific antibodies is commercially available by now. Thomm *et al.* (2018) use a commercial anti-TBEV ELISA IgG and IgM tests that employ inactivated TBEV antigens of strain K23 in addition to an indirect fluorescence assay based on Vero cells infected with POWV (Thomm, A.M., Schotthoefer, A.M., Dupuis, II, A.P., Kramer, L.D., Frost, H.M., Fritsche, T.R., Harrington, Y.A., Know, K.K., Kehl, S.C. (2018) Development and Validation of a Serologic Test Panel for Detection of Powassan Virus Infection in U.S. Patients Residing in Regions Where Lyme Disease Is Endemic, mSphere, 3(1), e00467-17). The authors report an overall test panel sensitivity of 89%.

Frost *et al.* (2017) performed a West Nile virus enzyme immunoassay, a Flavivirus mosaic panel and an IgG IFA assay panel including tests for TBEV, WNV, yellow fever virus, Dengue viruses 1-4, and Japanese encephalitis viruses on samples positive for POWV IgG by the IFA assay (Frost, H.M., Schotthoefer, A.M., Thomm A.M., Dupuis, II, A.P., Kehl, S.C., Kramer, L.D., Fritsche, T.R., Harrington, Y.A., Know, K.K. (2017) Serologic Evidence of Powassan Virus Infection in Patients with Suspected Lyme Disease, 23 (8), 1384-1388).

The state of the art discloses a range of diagnostic tests centering around the detection of antibodies to Flavivirus NS1 and GpE polypeptides from Flaviviruses other than POWV.

The Anti-West Nile Virus ELISA (IgM) (product no. EI 2662-9601 M) commercialized by EUROIMMUN Medizinische Labordiagnostika, Lübeck, Germany is based on the detection of WNV IgM antibodies to WNV GpE. The manufacturer's instructions disclose that significant cross reactivity with IgM to GpE from various other pathogens is an issue. For example, approximately 25 % of sera from patients suffering from Dengue virus infection were tested positive.

WO2017/144174 discloses the detection of antibodies to SEQ ID NO2, but not to SEQ ID NO1.

WO2017/174193 discloses a diagnostic carrier for the detection of ZIKV comprising a ZIKV GpE and a ZKV NS1 antigen. POWV, let alone reagents for the detection of POWV, are not disclosed. It is disclosed that ZKV GpE antigen shows a high cross reactivity towards antibodies to other Flaviviruses.

Tokarz *et al.* (2018) report a microarray-based test for the simultaneous detection of IgM and IgG antibodies to peptides comprising 12 amino acids derived from POWV GpE. The specificity and sensitivity of the assay, particularly compared to other flavivirus diagnostic assays, were not addressed. The purpose was to distinguish antibody responses to eight major tick-borne pathogens in the US, among them POWV, but no other flavivirus (Sci. Reports 8:3158, doi:10.1038/s41598-018-21349-2). The authors suggest that an ELISA is insufficiently reliable a diagnostic method and should therefore not be used.

Therefore, there is a long-standing need for the provision of a serological assay for the sensitive and specific diagnosis of POWV infection.

A problem underlying the present invention is to provide a serological assay for the diagnosis of a virus, preferably Flavivirus, more preferably POWV infection.

Another problem underlying the present invention is to provide a serological assay for the diagnosis of a virus, preferably Flavivirus, more preferably POWV infection, with excellent diagnostic reliability or increased diagnostically reliability relative to state of the art assays, in particular in terms of specificity and/or sensitivity. Optionally the assay is based on the detection of a single antibody.

Another problem underlying the present invention is to provide methods and reagents for such an assay.

The problem underlying the present invention is solved by the subject-matter of the attached independent dependent claims.

In a first aspect, the problem underlying the present invention is solved by a recombinant or chemically synthesized polypeptide comprising SEQ ID NO1 or a variant thereof, preferably in an isolated form.

In a second aspect, the problem underlying the present invention is solved by a diagnostically useful carrier comprising a means for specifically capturing an antibody to SEQ ID NO1, and/or a means for specifically capturing an antibody to SEQ ID NO2, in a sample from a subject.

In preferred embodiment, the diagnostically useful carrier is selected from the group comprising a bead, preferably a paramagnetic bead, a test strip, a microtiter plate, a membrane, preferably from the group comprising western blot, line blot and dot blot, a lateral flow device, a glass surface, a slide, a microarray, and a biochip.

In a third aspect, the problem is solved by a kit comprising the polypeptide or the diagnostically useful carrier, preferably further comprising a means for specifically detecting a captured antibody to SEQ ID NO1 and/or a means for specifically detecting a captured antibody to SEQ ID NO2.

In a preferred embodiment, the means for specifically detecting a captured antibody to SEQ ID NO1 is a secondary antibody recognizing IgM class antibodies.

In a preferred embodiment, the means for specifically detecting captured antibody to SEQ ID NO2 is a secondary antibody recognizing IgG or IgM, preferably IgG class antibodies.

In a preferred embodiment, the kit further comprises a recombinant or monoclonal antibody to SEQ ID NO2 and/or a recombinant or monoclonal antibody to SEQ ID NO1.

In a fourth aspect, the problem underlying the present invention is solved by a method for diagnosing, prognosing and monitoring the treatment of a virus, preferably Flavivirus, more preferably POWV infection comprising the step detecting in a sample from a subject the presence of an antibody to SEQ ID NO1 and/or an antibody to SEQ ID NO2, preferably SEQ ID NO1.

In a fifth aspect, the problem underlying the present invention is solved by a method for distinguishing a POWV infection from another viral infection, preferably with a Flavivirus other than POWV, comprising the step detecting in a sample from a subject the presence of an antibody to SEQ ID NO1 and/or an antibody to SEQ ID NO2, preferably SEQ ID NO1.

In a sixth aspect, the problem underlying the present invention is solved by a method comprising the step immobilizing on a diagnostically useful carrier a means for specifically capturing an antibody to SEQ ID NO1 and/or a means for specifically capturing an antibody to SEQ ID NO2, preferably a means for specifically capturing an antibody to SEQ ID NO1,
wherein the diagnostically useful carrier is preferably selected from the group comprising a bead, preferably a paramagnetic bead, a test strip, a microtiter plate, a membrane, preferably from the group comprising western blot, line blot and dot blot, a lateral flow device, a glass surface, a slide, a microarray and a biochip.

In a seventh aspect, the problem underlying the present invention is solved by a use of a polypeptide comprising SEQ ID NO1 and/or a SEQ ID NO2 or a variant thereof for the diagnosis of a Flavivirus, preferably POWV infection or for the manufacture of a kit for the diagnosis of a Flavivirus, preferably POWV infection, wherein preferably a POWV infection is distinguished from an infection with a Flavivirus other than POWV.

In a preferred embodiment the antibody to SEQ ID NO2 is an IgG or IgM, preferably IgG class antibody.

In a preferred embodiment, the antibody to SEQ ID NO1 is an IgM class antibody.

In an eighth aspect, the problem underlying the present invention is solved by a use of an IgM antibody to SEQ ID NO1 or a means for specifically capturing an IgM class antibody to SEQ ID NO1 for increasing the diagnostic reliability, preferably specificity of an immunoassay for the diagnosis of a virus, preferably Flavivirus, more preferably POWV infection.

The sample comprises antibodies from the patient, including the antibodies from the antibody classes to be detected, for example IgM or IgG class antibodies. Preferably it comprises a representative set of the entirety of the patient's antibodies. In another preferred embodiment, the antibodies from the class of antibodies to be detected are enriched, more preferably isolated. In a preferred embodiment, the sample is a blood, preferably plasma or serum or CSF sample, preferably a serum sample.

The present invention is based on the inventors' surprising finding that antibodies to SEQ ID NO1 and/or NS1 may be used for the sensitive and specific diagnosis of an infection, preferably with POWV. Moreover, the invention is based on the inventors' surprising finding that the presence of class IgM antibodies to SEQ ID NO1 is a significantly more sensitive and specific indicator of infection than antibodies to GpE homologues of other Flaviviruses.

According to the present invention, the polypeptide may be a recombinant protein, wherein the term "recombinant", as used herein, refers to a polypeptide produced using genetic engineering approaches at any stage of the production process, for example by fusing a nucleic acid encoding the polypeptide to a strong promoter for overexpression in cells or tissues or by engineering the sequence of the polypeptide itself. The person skilled in the art is familiar with methods for engineering nucleic acids and polypeptides encoded (for example, described in Sambrook, J., Fritsch, E. F. and Maniatis, T. (1989), Molecular Cloning, CSH or in Brown T. A. (1986), Gene Cloning - an introduction, Chapman & Hall) and for producing and purifying native or recombinant polypeptides (for example Handbooks "Strategies for Protein Purification", "Antibody Purification", published by GE Healthcare Life Sciences, and in Burgess, R. R., Deutscher, M. P. (2009): Guide to Protein Purification).

In a preferred embodiment, the polypeptide is POWV GpE, which has or comprises SEQ ID NO1 or A0A166AJ81 (Uniprot), preferably SEQ ID NO1. As throughout this application, any data base code used refers to the sequence available from said data base on the first priority date relating to said application. In another embodiment, POWV NS1 polypeptide may be used, which has or comprises SEQ DI NO2 from the present application or SEQ ID NO19 from WO2017/144174, preferably SEQ Dl NO2 from the present application. In another preferred embodiment, the polypeptide according to the present invention and used for the various embodiments of the present invention is an isolated polypeptide, wherein the term "isolated", as used herein, means that the polypeptide has been enriched compared to its state upon production using a biotechnological or synthetic approach and is preferably pure, *i.e.* at least 10, 20, 30, 40, 50, 60, 70, 80, 90, 95 or 99 percent of the polypeptide in the respective sample consists of said polypeptide as judged by SDS polyacrylamide gel electrophoresis followed by Coomassie blue staining and quantitative determination using a computer-aided densitometer.

In a preferred embodiment, the term "variant", as used herein, may refer to at least one fragment of the full length sequence referred to, for example SEQ ID NO1 or SEQ ID NO2, more preferably SEQ ID NO1, more specifically one or more amino acid or nucleic acid sequence which is, relative to the full-length sequence, truncated at one or both termini by one or more amino acids. Such a fragment comprises or encodes for a peptide having at least 10, 13, 15, 25, 50, 75, 100, 150, 200, 250 or 300 successive amino acids of the original sequence or a variant thereof. The total length of the variant may be at least 13, 25, 30, 40, 50, 60, 70, 80, 90, 100 or more amino acids.

In another preferred embodiment, the term "variant" relates not only to at least one fragment, but also a polypeptide or a fragment thereof comprising amino acid sequences, preferably a fragment comprising at least 25, more preferably 50, more preferably 200, more preferably 300, more preferably 350 successive amino acids, that are at least 40, 50, 60, 70, 75, 80, 85, 90, 92, 94, 95, 96, 97, 98 99, 99.4, 99.5, 99.6, 99.7, 99.8 or 99.9 % identical to the reference amino acid sequence referred to or the fragment thereof, wherein amino acids other than those essential for the biological activity, for example the ability to bind specifically to an antibody of interest, or the fold or structure of the polypeptide are deleted or substituted and/or one or more such essential amino acids are replaced in a conservative manner and/or amino acids are added or deleted such that the biological activity of the polypeptide is at least partially preserved. Known methods comprise various methods that may be used to align two given nucleic acid or amino acid sequences and to calculate the degree of identity, see for example Arthur Lesk (2008), Introduction to bioinformatics, Oxford University Press, 2008, 3rd edition. In a preferred embodiment, the ClustalW software (Larkin, M. A., Black-shields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007): Clustal W and Clustal X version 2.0. Bioinformatics, 23, 2947-2948) is used applying default settings. Exemplary variants of SEQ ID NO1 include the peptides EDLALPWKHKDNQD, DLALPWKHKDNQ, LALPWKHKDNQD, ALPWKHKDNQDW, LPWKHKDNQDWN and PWKHKDNQDWNS, which bind to IgM and IgG antibodies to SEQ ID NO1.

In a preferred embodiment, variants may, in addition, comprise chemical modifications, for example isotopic labels or covalent modifications such as glycosylation, phosphorylation, acetylation, decarboxylation, citrullination, hydroxylation and the like. The person skilled in the art is familiar with methods for the modification of polypeptides. Moreover, variants may also be generated by way of fusion with other known polypeptides or other variants.

The variant of the polypeptide has biological activity. In a preferred embodiment, such biological activity is the ability to bind to, preferably capture specifically the respective antibody if the variant is a variant of a sequence from the group comprising SEQ ID NO1, or SEQ ID NO2, preferably SEQ ID NO1. For example, a variant of SEQ ID NO1 has the ability to capture specifically an antibody to SEQ ID NO1 in a sample obtained from a subject suffering from or suspected of suffering from a viral infection, preferably POWV infection. Such variants have at least one epitope recognized by the antibody to be captured, for example one epitope in SEQ ID NO1 if an antibody to SEQ ID NO1 is captured. A variant of SEQ ID NO2 has the ability to capture specifically an antibody to SEQ ID NO2 in a sample from a subject suffering from or suspected of suffering from a viral infection, preferably POWV infection.

The person skilled in the art is capable of designing variants by starting from the original SEQ ID NO1 sequence, introducing modifications such as point mutations, truncations and the like and subsequently confirming that the variant still has biological activity by testing whether said variant binds to an antibody to SEQ ID NO1 in a sample obtained from a subject suffering from the disease to be diagnosed, preferably an infection, more preferably a viral infection, more preferably an infection with a Flavivirus, most preferably an infection with POWV. The 3D protein structure of homologues of SEQ ID NO1 or SEQ ID NO2 have been published and may be used for guidance in the design of variants and choice of the sequences that may be varied without compromising the biological activity and to distinguish them from important epitopes (for example Xu et al., Contribution of intertwined loop to membrane association revealed by Zika virus full-length NS1 structure (EMBO J., published on August 30, 2016, open access; Akey et al., Flavivirus NS1 structures reveal surfaces for associations with membranes and the immune system, Science 21;343(6173):881-5. doi: 10.1126/science; WO2015/095735). Variants may be identified by identifying naturally occurring fragments derived from the full-length protein or a precursor thereof, for example by purifying them using a polypeptide comprising SEQ ID NO1 or SEQ ID NO2, preferably SEQ ID NO1, as an affinity ligand followed by N-terminal Edman sequencing and/or tryptic digest in combination with mass spectrometry, and using them to practice the invention. Conservative amino acid substitutions may be used for all variants.

Within the scope of the present invention is a diagnostically useful carrier comprising a means for specifically capturing an antibody to an antigen such as SEQ ID NO1 or SEQ ID NO2, preferably SEQ ID NO1. In a preferred embodiment, the term "specifically capturing an antibody", as used herein, refers to the ability to bind specifically to the antibody of interest, preferably an IgA, IgM or IgG class antibody, to the effect that it is bound and may be removed from the sample, whereas other antibodies, preferably from the same class and/or to another antigen, are essentially not bound and remain in the sample. The antibody is preferably an antibody that binds to the antigen of interest only such as the one represented by SEQ ID NO1 or SEQ ID NO2, preferably SEQ ID NO1.

The diagnostically useful carrier according to the invention serves as a scaffold for the one or more means for specifically capturing an antibody, preferably a diagnostically relevant antibody to a Flavivirus antigen such as the one represented by SEQ ID NO1. Said carrier is suitable for carrying out a diagnostic method. By using a carrier rather than free, soluble means for specifically capturing an antibody, it is more straightforward to isolate and separate from the sample a complex comprising the means and the antibody and to wash said complex, for example for the purpose of removing any molecules binding non-specifically to the means, complex or carrier. In a preferred embodiment, the diagnostically useful carrier is a diagnostic device, preferably selected from the group comprising a bead, preferably a paramagnetic bead, a test strip, a microtiter plate, a blot and a membrane, and is preferably a line blot or microtiter plate, more preferably a microtiter plate.

The diagnostically useful carrier may be a slide, preferably glass or plastic slide for microscopy, comprising one or more eukaryotic cells, each spatially separated from each other and each expressing a polypeptide that is a means for specifically capturing an antibody. The one or more eukaryotic cells may be live cells, but are preferably fixed cells. State of the art protocols are available for fixing cells, for example using methanol or formaldehyde. Indirect immunofluorescence may be used to detect an antibody captured using such a carrier. One cell may comprise POWV GpE or a variant thereof. A cell may comprise POWV NS1 or a variant thereof. In addition, a cell may comprise a GpE protein from a Flavivirus other than POWV. In addition, a cell may comprise a GpE protein from a Flavivirus other than POWV. The cells may be arranged and the slide configured such that one drop of sample added is exposed to two or more cells at the same time, with barriers preventing cross-contamination in subsequent washing and developing reactions. The cells may be arranged and the carrier configured such that two or more cells may be examined simultaneously under a microscope.

The diagnostically useful carrier may be a bead configured for an immunoassay comprising a polypeptide comprising SEQ ID NO1 or a variant thereof. In a more preferred embodiment, the bead is a solid bead comprising carbohydrate such as sepharose or synthetic polymer such as latex, preferably a paramagnetic bead, which may be removed from a solution and concentrated, preferably at the surface of a vessel, by applying a magnetic field. The bead comprises a means for capturing an antibody linked to the bead by a covalent or non-covalent bond. A mixture of beads, for example one of which linked to SEQ ID NO1 or a variant thereof, and/or one linked to SEQ ID NO2 or a variant thereof, may be used.

In a preferred embodiment, the diagnostically useful device is a microtiter plate comprising a range of wells configured for an immunoassay such as an ELISA assay. Preferably the microtiter plate comprises a well coated with a means for specifically capturing an antibody to SEQ ID NO1 and or SEQ ID NO2, preferably SEQ ID NO1, which means is preferably a polypeptide comprising SEQ ID NO1 and or SEQ ID NO2 or a variant thereof. In a preferred embodiment, the term "microtiter plate" is a diagnostic device, preferably made from glass or plastic, more preferably plastic, comprising one or more, preferably more than one, more preferably at least 8 wells, in which reactions in liquid buffer may be run separately without cross-contamination. At least one of the wells is coated with a polypeptide, preferably an antigenic polypeptide that may be used to specifically capture a diagnostically useful antibody. If more than one means for specifically detecting an antigen is used, then preferably each means is in a well separate from other means. The microtiter plate may be used for running several samples in parallel, preferably in an automated fashion. The wells are preferably compatible with at least one routine detection techniques such colorimetry, immunofluorescence, detection of enzymatic activity, chemiluminscence, radioactivity or the like. In addition, a separate well may include one or more antigens for detecting another Flavivirus infection and may preferably comprise a means for capturing an antibody to a GpE from a Flavivirus other than POWV. A separate well may comprise a means for capturing an antibody to a NS1 from a Flavivirus other than POWV. A separate well comprise a Flavivirus other than POWV.

In a preferred embodiment, the term "specifically detecting a captured antibody", as used herein, means that the antibody binding specifically to the means for specifically capturing the antibody, preferably a polypeptide comprising SEQ ID NO1 or SED ID NO2, preferably SEQ ID NO1 or a variant thereof, following capture, binds specifically to the means for detecting the antibody, for example a secondary antibody. In a preferred embodiment, the term "specifically capturing an antibody", as used herein, means that the means for specifically capturing an antibody binds specifically to the antibody and does not bind, at a significant or detectable level to any other antibody. In a preferred embodiment, the term "binding specifically", as used herein, means that the binding is stronger than a binding reaction characterized by a dissociation constant of 1 x 10⁻⁵ M, more preferably 1 x 10⁻⁷ M, more preferably 1 x 10⁻⁸ M, more preferably 1 x 10⁻⁹ M, more preferably 1 x 10⁻¹⁰ M, more preferably 1 x 10⁻¹¹ M, more preferably 1 x 10⁻¹² M, as determined by surface plasmon resonance using Biacore equipment at 25°C in PBS buffer at pH 7.

In a preferred embodiment, the term "specifically detecting of an antibody", which antibody belongs to a certain class of antibodies, for example IgA, IgM or IgG class antibodies, means that said antibody is detected in an assay spatially separated from any binding reaction involving the detection of antibodies to the same antigen associated with *another* class and, more preferably, no antibody from another class against the same antigen is detected in the same reaction, for example an ELISA well. In other words, the assay is carried out such that the readout allows to distinguish between antibody classes, for example IgG or IgM antibodies from other antibody classes, and to conclude and detect specifically to which antibody class the detected antibody belongs or to detect only a specific class of antibody to an antigen of interest, preferably IgM antibodies to SEQ ID NO1 or IgG antibodies to SEQ ID NO2. This does not rule out that antibodies to the same antigen, but associated with another class of antibodies, may be detected in a spatially separate reaction simultaneously. For example, binding reactions involving the same antigen such as SEQ ID NO1 or SEQ ID NO2 or a variant thereof, may be run in separate wells of an ELISA micro-titerplate, but may be developed using secondary antibodies binding to different classes of antibodies, for example IgM and IgG, respectively.

In a preferred embodiment, an IgM class antibody to PWV GpE is detected. In a preferred embodiment, an IgG class antibody to PWV GpE is detected. In a preferred embodiment, an IgA class antibody to PWV GpE is detected. In a preferred embodiment, an IgM class antibody to PWV NS1 is detected. In a preferred embodiment, an IgG class antibody to PWV NS1 is detected. In a preferred embodiment, an IgA class antibody to PWV NS1 is detected. In a more preferred embodiment, an IgM antibody to GpE from a flavivirus other than PWV is detected in addition. In a more preferred embodiment, an IgG antibody to GpE from a flavivirus other than PWV is detected in addition. In a more preferred embodiment, an IgA antibody to GpE from a flavivirus other than PWV is detected in addition. In a more preferred embodiment, an IgM antibody to NS1 from a flavivirus other than PWV is detected in addition. In a more preferred embodiment, an IgG antibody to NS1 from a flavivirus other than PWV is detected in addition. In a more preferred embodiment, an IgA antibody to NS1 from a flavivirus other than PWV is detected in addition.

According to the present invention, a means for specifically detecting a captured antibody is provided or used to practice the present invention, optionally as part of a kit. In a preferred embodiment, the term "a means for specifically detecting a captured antibody", as used herein, refers to a reagent that binds specifically to the captured antibody, preferably to its constant region, such that the antibody captured or to be captured remains capable of binding to its antigen. The sequence of the constant region depends on the organism from which the sample is taken and analyzed, and the antibody class. Preferably, the means binds to human IgA, IgM or IgG class antibodies. If an antibody to SEQ ID NO1 is detected, it is preferred that the secondary antibody recognizes the constant region of IgM class antibodies. If an antibody to SEQ ID NO2 is detected, it is preferred that the secondary antibody recognizes constant region of IgG class antibodies.

In a more preferred embodiment, the means for specifically detecting a captured antibody is selected from the group comprising a secondary antibody, an aptamer and an anticalin (Skerra, A. (2008). "Alternative binding proteins: anticalins - harnessing the structural plasticity of the lipocalin ligand pocket to engineer novel binding activities". FEBS J. 275 (11): 2677-83) and is most preferably a secondary antibody. A secondary antibody may be a mammalian antibody, more preferably selected from the group comprising a cow, goat, chicken, rat, murine, porcine, equine or rabbit antibody. The means may comprise a detectable label, preferably from the group comprising an enzymatically active, colored label, preferably from the group comprising a gold and a latex label, chemiluminescent and fluorescent label.

If a polypeptide is used as the means for specifically capturing an antibody, said polypeptide, preferably comprising one or more sequences selected from the group comprising SEQ ID NO1 or SEQ ID NO2, preferably SEQ ID NO1, when used to carry out the teachings of the present invention, may be provided in any form and at any degree of purification, from tissues or cells comprising said polypeptide in an endogenous form, more preferably cells overexpressing the polypeptide, crude or enriched lysates of such cells, to purified and/or isolated polypeptide which may be essentially pure. In a preferred embodiment, the term "overexpressing", as used herein, means that the cell, preferably a eukaryotic, more preferably a mammalian or insect, more preferably a mammalian, more preferably a human cell, most preferably a HEK293 or HEK293T cell, has been genetically engineered such that it expresses more of the protein of interest than a non-engineered wild type cell would. In a preferred embodiment, the polypeptide is a native polypeptide, wherein the term "native polypeptide", as used herein, refers to a folded polypeptide comprising at least 15, 30, 50, 100 150, 200, 300 or 350 amino acids, preferably more than 30 amino acids, more preferably to a folded polypeptide purified from tissues or cells, more preferably from mammalian cells or tissues, optionally from non-recombinant tissues or cell. In another preferred embodiment, the polypeptide is a linear peptide having at least 7, more preferably at least 10 amino acid residues. If a native polypeptide is used, it is preferably enriched compared to its natural state. A recombinant polypeptide may comprise a C-terminal or N-terminal tag for affinity purification, immobilization or detection such as a His tag, as exemplified by SEQ ID NO 3 or SEQ ID NO4, or a streptavidin tag, preferably a streptavidin, which tag may preferably be removed by cleavage using a protease recognizing a protease cleavage site in a polypeptide linker between the tag and the N terminus or C-terminus, respectively, as part of the purification or method. The cleaved polypeptide may subsequently be attached to a diagnostically useful carrier to yield the diagnostically useful carrier according to the present invention. In another preferred embodiment, the means for specifically capturing an antibody is a POWV-infected eukaryotic, preferably human cell. Such a cell may be evaluated by fluorescence microscopy. The cells may be transiently or stably transfected, preferably transiently transfected, preferably with a vector comprising a sequence encoding SEQ ID NO1 or SEQ ID NO2, preferably SEQ ID NO2, under the control of an inducible or constantly induced promotor.

Said means for specifically capturing an antibody, together with the insoluble carrier to which it is attached, may be separated from a sample from a subject in a straightforward manner, for example by filtration, centrifugation, magnetism or decanting. Said means may be immobilized in a reversible or irreversible manner. For example, the immobilization is reversible if the means interacts with the carrier via ionic interactions which may be masked by addition of a high concentration of salt or if the molecule is bound via a cleavable covalent bond or a non-covalent bond. By contrast, the immobilization is irreversible if the means is tethered to the carrier via a covalent bond that cannot be cleaved in aqueous solution. The means may be indirectly immobilized, for example by immobilizing an antibody or other entity having affinity to the polypeptide, followed by addition of the means and formation of a means-antibody complex. A non-covalent bond may be made by chemically attaching a ligand to the carrier, preferably via a covalent bond, and fusing to the means a polypeptide having affinity to the ligand. In a preferred embodiment, the ligand is selected from the group comprising biotin, in which case the polypeptide having affinity may be streptavidin or a variant thereof binding to biotin, glutathione (polypeptide having affinity: glutathione-S-transferase), Nickel (polypeptide having affinity: His tag), Flag tag (polypeptide having affinity: anti-flag antibody), carbohydrate such as maltose or cellulose (polypeptide having affinity: maltose or cellulose binding protein), and is preferably biotin.

According to the present invention, a nucleic acid encoding the polypeptide according to the present invention such as a polypeptide comprising SEQ ID NO1 or SEQ ID NO2, preferably SEQ ID NO1 or a variant thereof, optionally with an inducible promotor, which polypeptide is preferably for use for the diagnosis of a disease or the manufacture of a kit or reagent for such use, is provided. Said nucleic acid may be a part of a vector, preferably for expressing said nucleic acid. A eukaryotic or prokaryotic, preferably eukaryotic cell comprising this vector and preferably expressing the polypeptide encoding by the vector, is also provided. The nucleic acid, the vector and the cell may be used for the manufacture of a kit for use according to the present invention such as use of an antibody to SEQ ID NO1 or SEQ ID NO2, preferably SEQ ID NO1. The nucleic acid may be expressed, the polypeptide encoded purified and used, preferably immobilized on a diagnostically useful carrier, in order to make the diagnostically useful carrier according to the present invention. The nucleic acid may be used to design and prepare a nucleic acid construct encoding a variant of the polypeptide for expression and use of the resulting variant for the preparation of a diagnostically useful carrier and for the detection of the respective antibody such as an antibody to SEQ ID NO1 or SEQ ID NO2, preferably SEQ ID NO1.

The inventive teachings provide a kit, preferably for diagnosing an infection, more preferably for diagnosing a flavivirus infection, most preferably a POWV infection. Such a kit is a container that comprises specific reagents required to practice the inventive method, in particular the diagnostically useful carrier according to the present invention, optionally in addition to one or more reagents and solutions required to practice the inventive method, preferably selected from or all from the group comprising sample dilution buffer, washing buffer and a means for detecting any specifically captured antibody and optionally a means for detecting the specifically captured antibody, which may optionally be attached to the secondary antibody, for example a fluorescent, enzymatically active, radioactive, chemiluminescent, preferably electrochemiluminscent label or a spin label. The kit may comprise a chemical solution for carrying out a detection reaction such as 3,3',5,5'-tetramethylbenzidine, *p*-Nitrophenyl Phosphate, 2,2'-Azinobis [3-ethylbenzothiazoline-6-sulfonic acid or o-phenylenediamine dihydrochloride for a colorimetric reaction tripropylamin for an electrochemiluminescence reaction. Furthermore, it may comprise instructions detailing how to use the kit and the inventive diagnostically useful carrier for contacting the inventive polypeptide with a bodily fluid sample from a subject, preferably a human subject.

Furthermore, the kit may comprise a positive control, for example a recombinant antibody known to bind to SEQ ID NO1 and/or a recombinant antibody known to bind to SEQ ID NO2, and a negative control, for example a protein having no detectable affinity to SEQ ID NO1 or to SEQ ID NO2. Finally, the kit may comprise one or more calibrators, which is preferably a solution comprising a SEQ ID NO1-binding and/or SEQ ID NO2-binding, preferably SEQ ID NO1-binding antibody for preparing a calibration curve. In a more preferred embodiment, the kit comprises more than one, preferably three or more calibrators comprising different concentrations of an antibody to SEQ ID NO1, which antibody is preferably recognized by a secondary antibody to human IgM class antibodies. In a more preferred embodiment, the kit comprises at least one antibody to SEQ ID NO2, which antibody is preferably recognized by a secondary antibody to human IgG class antibodies. Such calibrator may be or comprise a chimeric antibody comprising a human constant region and optionally a variable region from a mammal other than a human. If more than one calibrator is used, the two or more calibrators comprise different concentrations of the antibody, preferably at a range of concentrations that allow the user to set up a calibration curve.

If IgM class antibodies binding to SEQ ID NO1 or SEQ ID NO2, preferably SEQ ID NO1 are detected, IgG class antibodies may be removed or their concentration may be decreased prior to determining the IgM class antibodies. This may be achieved by pre-absorbing IgG class antibodies, for example by contacting them with an anti-IgG antibody, for example a mammalian, preferably goat anti-human IgG antibody. This antibody may absorb IgG antibodies that may interfere with the IgM detection assay. Suitable reagents are commercially available, for example EUROSORB commercialized by EUROIMMUN, Lübeck. Alternatively, this may be achieved by isolating the entirety of IgM class antibody prior to detecting an IgM antibody to SEQ ID NO1 or SEQ ID NO2, preferably SEQ ID NO1.

For therapeutic purposes, a vaccine may be provided that comprises the polypeptide comprising SEQ ID NO1 and/or the polypeptide comprising SEQ ID NO2, preferably SEQ ID NO1 or a variant thereof may be formulated with one or more diluents, one or more glidants, and/or one or more filling agents. The preparation of suitable formulations is described in the state of the art, for example in US20130022631A1. The vaccine formulation may comprise, in addition to the purified polypeptide, a pharmaceutically accepted buffer such as phosphate or phosphate-citrate buffer in the pH range 6.4-7.5 with added stabilizing agents that may include one or more of the following, but is not limited to: human serum albumin, gelatin, reducing and non-reducing sugars, amino acids, polyols such as sorbitol and mannitol, glycerol organic and inorganic salts, polyvinyl pyrrolidone etc. The stable formulation may be in a liquid form is suitable for intramuscular/intradermal/subcutaneous/intravenous administration in a human host. The stable formulation may be in a dry lyophilized form can be reconstituted with a suitable solvent before administration. The formulations may be suitable for oral and intranasal administration in humans.

The invention provides a pharmaceutical composition or a vaccine, which composition or immunogenic composition such as a vaccine comprises a polypeptide comprising SEQ ID NO1 and/or SEQ ID NO2, preferably SEQ ID NO1 or a variant thereof An immunogenic composition or vaccine may comprise components to inactivate a virus or bacteria and stabilize the vaccine, helping to preserve the vaccine and prevent it from losing its potency over time. Adjuvants are added to vaccines to simulate the production of antibodies against the vaccine to make it more effective. An adjuvant could be organic or inorganic. The most common inorganic adjuvants for human vaccines include aluminum phosphate and aluminum hydroxide. Organic adjuvants could be based on the organic compound squalene and an oil [squalene] in water adjuvant can be used.

An immunogenic composition may comprise stabilizers that help the vaccine to maintain its effectiveness during storage, e.g., MgCl₂, MgSO₄, lactose-sorbitol, or sorbitol-gelatin, and preservatives to prevent bacterial and fungal growth, e.g., thiomersal, formaldehyde, or phenol derivatives, antibiotics. The composition is preferably suitable for administration to a subject, preferably a mammalian subject, more preferably to a human. Such a pharmaceutical composition may comprise a pharmaceutically acceptable carrier. The pharmaceutical composition may, for example, be administered orally, parenterally, by inhalation spray, topically, by eyedrops, rectally, nasally, buccally, vaginally or via an implanted reservoir, wherein the term "parentally", as used herein, comprises subcutaneous, intracutaneous, intravenous, intramuscular, intra-articular, intrasynovial, instrasternal, intrathecal, intralesional and intracranial injection or infusion techniques. The pharmaceutical composition may be provided in suitable dosage forms, for example capsules, tablets and aqueous suspensions and solutions, preferably in sterile form. It may be used in a method of treatment of a disease, which method comprises administering an effective amount of the inventive polypeptide to a subject.

The inventive method for diagnosing a Flavivirus infection, preferably for distinguishing a primary from a secondary Flavivirus infection, may comprise the step detecting in a first sample from a subject an IgA class antibody to NS1 of a Flavivirus, preferably POWV, optionally further comprising detecting in said first sample an IgM and/or IgG class, preferably IgM class antibody to NS1 of a Flavivirus, preferably POWV, such as SEQ ID NO2 or a variant thereof. In a preferred embodiment, the method further comprises the step detecting in a second sample obtained from said subject an IgA class antibody to NS1 of said Flavivirus, optionally further comprising detecting in said second sample an IgM and/or IgG class, preferably IgM class antibody to NS1 of a Flavivirus such as SEQ ID NO2, optionally further comprising detecting in said first sample an IgM and/or IgG class, preferably IgM class antibody to NS1 of a Flavivirus, more preferably SEQ ID NO2.

A dynamic titer of IgA or IgM, preferably IgA class antibodies to NS1 of a Flavivirus, preferably to SEQ ID NO2, increasing and decreasing significantly relatively to the background before emergence of IgG class antibodies (i.e. seroconversion), may indicate an acute POWV infection, which is a primary Flavivirus infection. By contrast, a parallel increase of IgA and IgG (albeit the latter at higher levels) may indicate an acute POWV infection, which is a secondary Flavivirus infection. In a preferred embodiment, the first sample is obtained at least 3, 4, 5, 6 days, 1, 2 3 or 4 weeks following the subject's exposure or suspected exposure to a Flavivirus. In a preferred embodiment, the first sample is taken in the two weeks after onset of symptoms. The presence or absence of antibodies may be determined as well as their relative levels over time. The second sample may be obtained at least 3, 4, 5, 6 days, 1, 2, 4, 6, 8, 12, 16, 20, 24, 28 or 32 weeks later than the first sample, preferably at least 3 days, more preferably at least 7 days. A total number of at least 2, 3, 4, 5 or six samples may be taken, preferably at least 2 samples, optionally each sample at least 1 day, 3 days, one week, preferably one week after the previous sample. The total concentrations of IgG, IgM and/or IgA, preferably IgM, may be determined in addition, for example to rule out insufficiencies. This way, the titers of the respective antibodies may be monitored over time.

In a preferred embodiment, the titer of IgM, IgG and/or IgA, preferably IgA and IgG, more preferably IgA to NS1, preferably to SEQ ID NO2 is monitored by detecting the presence or absence or, preferably relative level over time for a period of at least 3, 4, 5, 6, 10, 14, 21, 28, 35 or 42 days, preferably at least 6 days, with the first sample being taken at least five days, preferably at least 7 days following onset of the symptoms. Seroconversion may be detected by monitoring the presence or absence or relative level over time of IgG class antibodies to NS1, preferably SEQ ID NO2. This may help identify the time window in which the increase and decrease of IgM and/or IgA class antibodies would be expected or concluding that this time window has passed.

The inventive method, kit and carriers may be used to distinguish between a primary and a secondary Flavivirus infection, preferably POWV infection. In a preferred embodiment, the term "primary infection", as used herein, refers to an infection of a person who has never had an infection with said Flavivirus or another Flavivirus, preferably said Flavivirus, more preferably the POWV, by contrast to a secondary infection in a patient who has been exposed to a virus or immunogenic compositions derived thereof before. In a preferred embodiment, this may involve distinguishing a primary POWV infection from a secondary infection with another Flavivirus.

A Flavivirus other than POWV may be selected from the group comprising dengue virus 1, dengue virus 2, dengue virus 3, dengue virus 4, Yellow fever virus, Tick-borne encephalitis virus, Usutu virus, West Nile virus, Zika virus and Japanese encephalitis virus, or may refer to two or more viruses from this group, preferably all of them.

In addition, the presence of an antibody to an NS1 from a Flavivirus other than POWV may be detected or the diagnostically useful carrier may comprise a means for capturing an antibody to an NS1 from a Flavivirus other than POWV. An NS1 protein from a Flavivirus other than POWV may be selected from the group comprising Zika NS1 (SEQ ID NO1 from WO2017/144174), dengue virus 1 NS1 (SEQ ID NO2 from WO2017/144174), dengue virus 2 NS1 (SEQ ID NO3 from WO2017/144174), dengue virus 3 NS1 (SEQ ID NO4 from WO2017/144174), dengue virus 4 (NS1 SEQ ID NO5 from WO2017/144174), West Nile virus NS1 (SEQ ID NO6 from WO2017/144174), Tick-borne encephalitis virus NS1 (SEQ ID NO7 from WO2017/144174), Japanese encephalitis virus NS1 (SEQ ID NO8 from WO2017/144174) and Yellow fever virus NS1 (SEQ ID NO9 from WO2017/144174) or a variant thereof.

In addition, the presence of an antibody to a GpE from a Flavivirus other than POWV may be detected or the diagnostically useful carrier may comprise a means for capturing an antibody to a GpE from a Flavivirus other than POWV. An GpE protein from a Flavivirus other than POWV may be selected from the group comprising Zika GpE (SEQ ID NO11 from WO2017/144174), dengue virus 1 GpE (SEQ ID NO12 from WO2017/144174), dengue virus 2 GpE (SEQ ID NO13 from WO2017/144174), dengue virus 3 GpE (SEQ ID NO14 from WO2017/144174), dengue virus 4 GpE (SEQ ID NO15 from WO2017/144174) West Nile virus GpE (SEQ ID NO16 from WO2017/144174), Tick-borne encephalitis virus GpE (SEQ ID NO17 from WO2017/144174), Japanese encephalitis virus GpE (SEQ ID NO18 from WO2017/144174) and Yellow fever virus GpE (Uniprot Q6DV88) or a variant thereof.

In many cases detecting or detecting the presence of an antibody, optionally meaning determining whether the concentration of the antibody is beyond a certain threshold preferably as set by measurement using ELISA, preferably as described in Example 1, in the implicit detection limit by this method, often suggested by the detection limit, in the sample, is sufficient for the diagnosis. If the antibody can be detected, this will be information instrumental for the clinician's diagnosis and indicates an increased likelihood that the patient suffers from a disease. In a preferred embodiment, the relative concentration of the antibody in the serum, compared to the level that may be found in the average healthy subject, may be determined. In a preferred embodiment, the term "detecting the presence", as used herein, means that it is sufficient to check whether a signal sufficiently beyond any background level may be detected using a suitable complex detection method that indicates that the antibody of interest is present or more antibody of interest is present than would be in a healthy subject. In a more preferred embodiment this may involve determining whether the concentration is at least 0.1, preferably 0.2, 0.5, 1, 2, 5, 10, 20, 25, 50, 100, 200, 500, 1000, 10000 or 100000 times higher than the concentration of the antibody of interest found in the average healthy subject.

In a preferred embodiment, the term "diagnosis", as used herein, refers to any kind of procedure aiming to obtain information instrumental in the assessment whether a patient suffers or is likely or more likely than the average or a comparative subject, the latter preferably having similar symptoms, to suffer from a certain disease or disorder in the past, at the time of the diagnosis or in the future, to find out how the disease is progressing or is likely to progress in the future or to evaluate the responsiveness of a patient with regard to a certain treatment, for example the administration of suitable drugs such as drugs for the desensitization of allergic patients. In other words, the term "diagnosis" comprises not only diagnosing, but also prognosticating and/or monitoring the course of a disease or disorder.

Therefore, the term "diagnosis" does preferably not imply that the diagnostic methods or agents according to the present invention will be definitive and sufficient to finalize the diagnosis on the basis of a single test, let alone parameter, but may refer to a contribution to what is referred to as a "differential diagnosis", *i.e.* a systematic diagnostic procedure considering the likelihood of a range of possible conditions on the basis of a range of diagnostic parameters. The term "diagnosis" may also refer to a method or agent used to choose the most promising treatment regime for a patient. In other words, the method or agent may relate to selecting a treatment regimen for a subject.

The invention may be used to provide a prognosis whether a pregnant woman's newborn child is likely to suffer from a deformity if the sample from the pregnant woman is tested. Preferably, the pregnant woman may have symptoms suggesting that she may suffer from a flaviviral infection or may very actually suffer from an infection.

The present invention relates to a method comprising the step detecting in a sample from a subject the presence or absence of an antibody to an antigenic polypeptide such as a polypeptide comprising a SEQ ID NO1 or SEQ ID NO2, preferably SEQ ID NO1 or a variant thereof. This method preferably comprises immobilizing said antibody followed by specific detection of said antibody, for example by way of the steps a) (optionally) providing a sample from a subject, b) contacting the sample with the diagnostically useful carrier according to the present invention under conditions compatible with the formation of a complex comprising the diagnostically useful carrier and the antibody, more specifically the means for specifically capturing the antibody and the antibody, c) isolating any said complex, for example by removing the sample, d) optionally washing said complex, and e) optionally detecting said complex. The method is preferably an *in vitro* method. The detection of the complex for the prognosis, diagnosis, methods or test kit according to the present invention comprises the use of a method selected from the group comprising immunodiffusion techniques, immunoelectrophoretic techniques, light scattering immunoassays, agglutination techniques, labeled immunoassays such as those from the group comprising radiolabeled immunoassay, enzyme immunoassays such as colorimetric assays, chemiluminscence, preferably electrochemiluminescence, immunoassays and immunofluorescence techniques. The person skilled in the art is familiar with these methods, which are also described in the state of the art, for example in Zane, H. D. (2001): Immunology - Theoretical & Practical Concepts in Laboratory Medicine, W. B. Saunders Company, in particular in Chapter 14. The method may further involve testing the avidity of antibodies to SEQ ID NO1 or SEQ ID NO2 in the sample, preferably of antibodies to SEQ ID NO1.

The present invention is further illustrated by the following examples, sequences and figures from which further features, embodiments, aspects and advantages of the present invention may be taken. All methods and materials similar or equivalent to those described herein can be used in the practice or testing of the present invention, with suitable methods and materials being described herein. All publications, patent applications, patents, and other references mentioned herein are incorporated by reference in their entirety. Further, the materials, methods, and examples are illustrative only and are not intended to be limiting, unless otherwise specified.
**SEQ ID NO 1 (mature sequence of POWV Glycoprotein E)** 
ASMRCTHLENRDFVTGTQGTTRVSLVLELGGCVTITAEGKPSIDVWLEDIFQESPAETREYC
LHAKLTNTKVEARCPTTGPATLPEEHQANMVCKRDQSDRGWGNHCGFFGKGSIVACAKFE
CEEAKKAVGHVYDSTKITYVVKVEPHTGDYLAANETNSNRKSAQFTVASEKVILRLGDYGDV
SLTCKVASGIDVAQTVVMSLDSSKDHLPSAWQVHRDWFEDLALPWKHKDNQDWNSVEKL
VEFGPPHAVKMDVFNLGDQTAVLLKSLAGVPLASVEGQKYHLKSGHVTCDVGLEKLKLKGT
TYSMCDKAKFKWKRVPVDSGHDTVVMEVSYTGSDKPCRIPVRAVAHGVPAVNVAMLITPN
PTIETNGGGFIEMQLPPGDNIIYVGDLSQQWFQKG
**SEQ ID NO 2 (mature sequence of POWV NS1)** 
GHMDYGCAIDPERMEIRCGEGLVVWKEVSEWYDGYAYHPESPDTLAQALREAFERGVCG
VVPQNRLEMAMWRSTAPELNLVLSEGEANLTIVVDKTDPADYRGGTPMVLKKTGKESKVS
WKSWGKSILWSVPDSPRRMMMGVDGVGECPLYRRATGVFTVAEFGVGLRTKVFLDLRGE
ASKECDTGVMGAAVKNGKAIHTDQSMWMSSFRNDTGTYIHELILTDLRNCTWPASHTIDND
GVLDSHLFLPVTLAGPRSKYNRIPGYSEQVRGPWDQTPLRVVRDHCPGTSVRIDSHCDKR
GASVRSTTESGKIIPEWCCRACELPPVTFRSGTDCWYAMEIRPVHSQGGLVRSMVVALEVT
WGNNEPYKYWPQLSTNGTAHGHPHEIILYYYELYPTMTVVVVSVATFILLSMVGMAAGMCM
CARRRCITPYELTPGATVPFLLSLICCIRTAKA
**SEQ ID NO 3 (His-tagged POWV Glycoprotein E as used in examples)** 
ASMRCTHLENRDFVTGTQGTTRVSLVLELGGCVTITAEGKPSIDVWLEDIFQESPAETREYC
LHAKLTNTKVEARCPTTGPATLPEEHQANMVCKRDQSDRGWGNHCGFFGKGSIVACAKFE
CEEAKKAVGHVYDSTKITYVVKVEPHTGDYLAANETNSNRKSAQFTVASEKVILRLGDYGDV
SLTCKVASGIDVAQTVVMSLDSSKDHLPSAWQVHRDWFEDLALPWKHKDNQDWNSVEKL
VEFGPPHAVKMDVFNLGDQTAVLLKSLAGVPLASVEGQKYHLKSGHVTCDVGLEKLKLKGT
TYSMCDKAKFKWKRVPVDSGHDTVVMEVSYTGSDKPCRIPVRAVAHGVPAVNVAMLITPN
PTIETNGGGFIEMQLPPGDNIIYVGDLSQQWFQKGLEHHHHHHHH
**SEQ ID NO 4 (His-tagged POWV NS1 as used in examples)** 
GHMDYGCAIDPERMEIRCGEGLVVWKEVSEWYDGYAYHPESPDTLAQALREAFERGVCG
VVPQNRLEMAMWRSTAPELNLVLSEGEANLTIVVDKTDPADYRGGTPMVLKKTGKESKVS
WKSWGKSILWSVPDSPRRMMMGVDGVGECPLYRRATGVFTVAEFGVGLRTKVFLDLRGE
ASKECDTGVMGAAVKNGKAIHTDQSMWMSSFRNDTGTYIHELILTDLRNCTWPASHTIDND
GVLDSHLFLPVTLAGPRSKYNRIPGYSEQVRGPWDQTPLRVVRDHCPGTSVRIDSHCDKR
GASVRSTTESGKIIPEWCCRACELPPVTFRSGTDCWYAMEIRPVHSQGGLVRSMVVALEVT
WGNNEPYKYWPQLSTNGTAHGHPHEIILYYYELYPTMTVVVVSVATFILLSMVGMAAGMCM
CARRRCITPYELTPGATVPFLLSLICCIRTAKALEHHHHHHHH

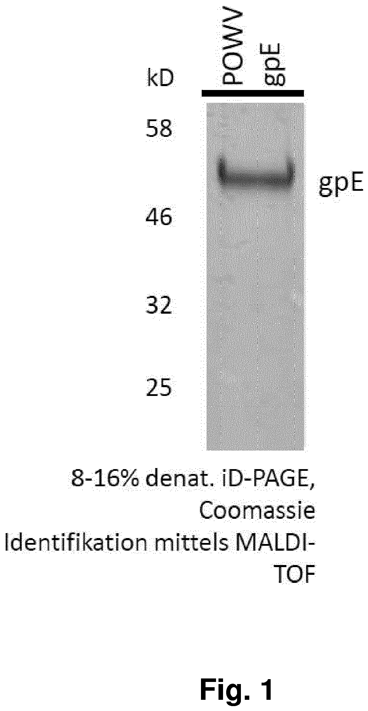

   **Fig. 1** shows SDS-PAGE and Coomassie staining of 1 µg purified recombinant GpE. 1 µg protein was separated on an 8-16 % denaturing iD-PAGE gel, documenting high protein purity. Molecular weight markers are indicated on the left.

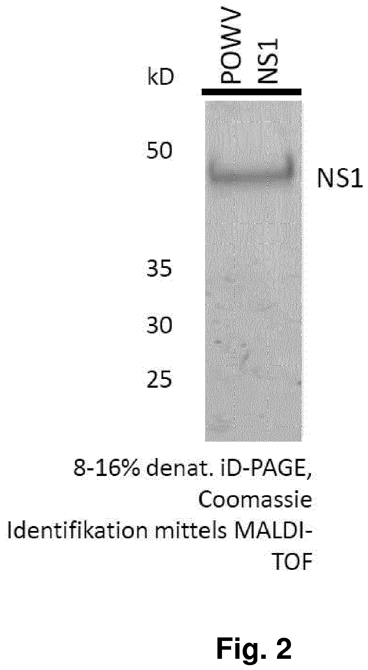

   **Fig. 2** shows SDS-PAGE and Coomassie staining of 1 µg purified recombinant NS1. 1 µg protein was separated on an 8-16 % denaturing iD-PAGE gel, documenting high protein purity. Molecular weight markers are indicated on the left.
   **Fig. 3** shows the results of an ELISA-based assay for the detection of POWV glycoprotein E and NS1 IgM. Anti-POWV positive samples (●), samples from healthy blood donors (x), Anti-DENV positive samples (▲), Anti-WNV positive samples (★) and Anti-ZIKV positive samples (■) were included. The broken line defines a borderline range (Ratio 0.8 - 1.1).
   **Fig. 4** shows the results of an ELISA-based assay for the detection of POWV glycoprotein E and NS1 IgG. Anti-POWV positive samples (●), samples from healthy blood donors (x), Anti-DENV positive samples (▲), Anti-WNV positive samples (★) and Anti-ZIKV positive samples (■) were included. The broken line defines a borderline range (Ratio 0.8 - 1.1).

### Example 1: POWV GpE Protein expression and purification

Recombinant POWV GpE was expressed in Sf9 cells using standard cloning and expression methods based on the pHEP plasmid with an artificial signal sequence and a C-terminal His tag (SEQ ID NO1). Transfected cells were cultures at 27,5°C in Insect-XPRESS™ Protein-free Insect Cell Medium with L-glutamine for four days. Cells were harvested, resuspended in 20 mM Tris-HCI pH 7.4, 10% (w/v) sucrose, 5 mM EDTA, 1 mM PMSF and stored at - 80°C until further use.

To prepare GpE, cell culture supernatant was adjusted to 11 mmol/l MOPS pH7,0, 200 mmol/l sodium chloride, 50 mmol/l magnesium chloride, 20 mmol/l imidazole, cleared by centrifugation for 30 minutes at 17,600xg, 4°C, applied to Nickel Rapid Run (Agarose Bead Technologies, Miami, FL, USA) equilibrated with 10 mmol/l MOPS pH 7.0, 300 mmol/l sodium chloride, 20 mmol/l imidazole and eluted by increasing the imidazole concentration to 150 mmol/l. All fractions containing GpE[POWV] were pooled and concentrated by ultrafiltration (Vivaspin, Sartorius, Göttingen, Germany). The final preparation was stored at -80°C until further use.

The final protein preparation of GpE were treated with or without 16 mmol/l dithiotreitol and incubated at 70°C or at room temperature for 10 minutes, followed by SDS gel electrophoresis and Coomassie staining.

When separated by SDS-PAGE, GpE migrated essentially according to its predicted molecular mass (49 kDa; **Fig**. **1**). Protein identity was verified by mass spectrometry.

### Example 2: POWV NS1 Protein expression and purification

Recombinant POWV NS1 was expressed in HEK293T cells using standard cloning and expression methods based on the pTriEx-1 plasmid with an artificial signal sequence and a C-terminal His tag (SEQ ID NO 2). Transfected cells were cultures at 37°C and 8.5% CO2 in Dulbecco's modified eagle's medium with 10% fetal calf serum, 100 U/ml penicillin and 0.1 mg/ml streptomycin for three to five days. Cells were harvested, resuspended in 20 mM Tris-HCI pH 7.4, 10% (w/v) sucrose, 5 mM EDTA, 1 mM PMSF and stored at -80°C until further use.

To prepare NS1, cell culture supernatant was adjusted to 5 mmol/l tris chloride pH 8.0, 164 mmol/l sodium chloride, 50 mmol/l magnesium chloride, 20 mmol/l imidazole, 0,1% Triton X-100, cleared by centrifugation for 30 minutes at 17,600xg, 4°C, applied to Nickel Rapid Run (Agarose Bead Technologies, Miami, FL, USA) equilibrated with 5 mmol/l tris chloride pH 8.0, 300 mmol/l sodium chloride, 20 mmol/l imidazole and eluted by increasing the imidazole concentration to 150 mmol/l. All fractions containing NS1[POWV] were pooled and concentrated by ultrafiltration (VivaSpin, Sartorius, Göttingen, Germany). The final preparation was stored at -80°C until further use.

The final protein preparation of NS1 was treated with or without 16 mmol/l dithiotreitol and incubated at 70°C or at room temperature for 10 minutes, followed by SDS gel electrophoresis and Coomassie staining.

When separated by SDS-PAGE, NS1 migrated essentially according to its predicted molecular mass (43.2 kDa; **Fig**. **2**). Protein identity was verified by mass spectrometry.

### Example 3 and 4: ELISA-based assay for the detection of POWV glycoprotein E IgM and ELISA-based assay for the detection of POWV NS1 IgG

The following experiments were performed to evaluate the performance of different Powassan virus antigens in an indirect ELISA for the detection of specific anti-POWV virus antibodies in human serum or plasma, more specifically PWS GpE and NS1 as prepared in Examples 1 and 2, respectively.

### Samples

Panel 1 (sensitivity panel): Eight clinical samples (7 serum samples; 1 CSF sample) of patients with acute POWV infection.
Panel 2 (specificity panel): 171 samples which originate from healthy individuals. Previous exposure to POWV is highly unlikely because of the origin of the samples (Germany) and the rare occurrence of infections even in endemic regions.
Panel 3 (cross reactivity panel): This panel consists of 40 samples positive for antibodies against Dengue virus, 17 samples positive for antibodies against West Nil virus and 39 samples positive for antibodies against Zika virus. This panel will be used for determination of cross-reactivity of the antigens used.

### 1. Preparation of coated microtiter plates:

Two different POWV antigens were used: glycoprotein E (GpE; prepared from cell culture supernatant of Sf9 cells) and non-structural protein 1 (NS1; prepared from cell culture supernatant of HEK293 cells).

For use in microtiter ELISA these antigens were diluted in PBS to final concentrations of 2.5 µg/ml, respectively. Microtiter plates were coated with 100 µl of antigen dilution per well.

### 2. Anti-POWV IgM ELISA:

All reagents used during this experiment are included in every EUROIMMUN IgM ELISA Test-Kit for infectious diagnostics (e.g. EI 2668-9601 M). Sera were diluted 1:101 in IgM sample buffer containing IgG/RF absorbent and incubated at room temperature for 10 min to absorb rheumatoid factors and IgG. Samples were applied to microtiter plates and incubated as described for commercial EUROIMMUN ELISA Test-Kits (e.g. EI 2668-9601 M). In brief: 60 min at 37 °C; 3 washing steps using EUROIMMUN wash buffer; addition of 100 µl of peroxidase-labelled anti-human IgM conjugate (goat) per well; incubation for 30 min at room temperature; 3 washing steps using EUROIMMUN wash buffer; addition of 100 µl of chromogen/substrate solution (TMB/H2O2) per well; incubation for 15 min at room temperature; addition of 100 µl stop-solution (0.5 M sulfuric acid); measurement of optical density at 450 nm.

Panel 1 was used for evaluation of sensitivity of the respective antigens for detection of specific anti-POWV IgM antibodies. Panel 2 and Panel 3 were incubated to determine specificity and cross reactivity of the test system.

### 3. Anti-POWV IgG ELISA:

All reagents used during this experiment are included in every EUROIMMUN IgG ELISA Test-Kit for infectious diagnostics (e.g. EI 2668-9601 G). Sera were diluted in IgG sample buffer and diluted samples were applied to microtiter plates and incubated as described for the anti-POWV IgM ELISA.

Panel 1 was used for evaluation of sensitivity of the respective antigens for detection of specific anti-POWV IgG antibodies. Panel 2 and Panel 3 were incubated to determine specificity and cross reactivity of the test system.

### Results

### 1. Sensitivity and specificity of anti-POWV IgM ELISA

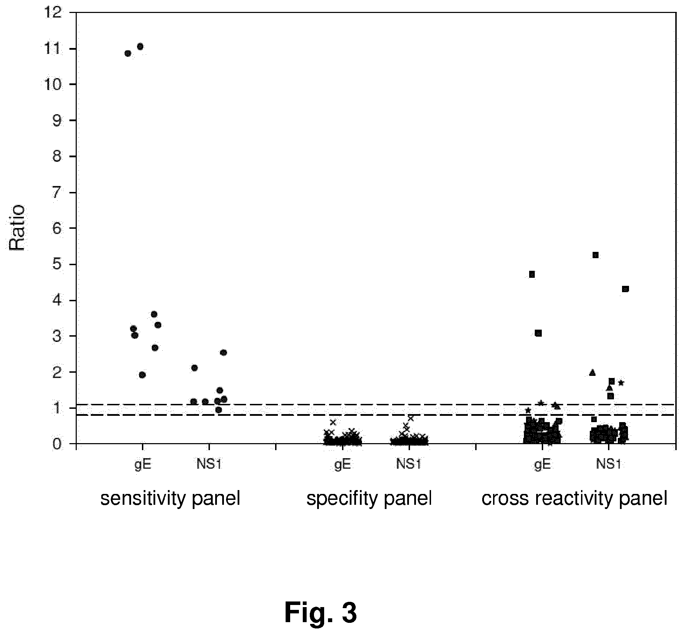

Comparison of GpE and NS1 antigens for the detection of specific anti-POWV IgM antibodies showed high sensitivity for ELISA using either of the antigens (GpE: 100.0 % [8/8]; NS1: 87.5% [7/8]) (Fig. 3). Nevertheless, usage of GpE led to higher reactivity of positive samples suggesting higher sensitivity when used for screening. There were no advantages achieved by using NS1 with regard to specificity (98.2% [NS1] vs. 97.1% [GpE]) and cross reactivity (Fig. 3). In contrast, cross reactivity was lower for the GpE -based ELISA than for the NS1-based ELISA (DENV: 5,0% vs. 0,0%; WNV: 5,9% vs. 5,9%; ZIKV: 10,3% vs. 5,1%).

### 2. Sensitivity and specificity of anti-POWV IgG ELISA

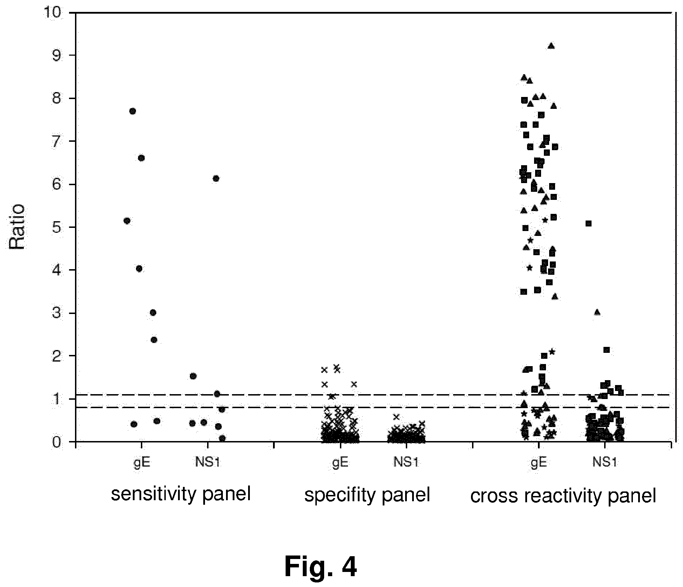

Comparison of GpE and NS1 antigens showed sensitivities of 75.0% and 37.5%, respectively. The sample set used for evaluation of sensitivity of this test are samples originating from patients with acute infections. If these samples were drawn at early time points of infection there is the possibility of lacking anti-Powassan IgG because of seroconversion has not taken place yet. GpE suggests to be more sensitive for the detection of IgG, but due to higher cross reactivity of GpE in comparison to NS1 (Fig. 4), IgG antibodies measured may result from earlier flavivirus contact or vaccination (e.g. YFV vaccination).

The NS1-based anti-POWV IgG ELISA showed a specificity of 95.3% in panel 2 (healthy blood donors), in contrast to 87,9% for the GpE based ELISA. Again the lower specificity of GpE may result from prevalence of antibodies directed against other flaviviruses.

The higher specificity of the NS1-based ELISA was strengthened by drastic reduction of cross reactivity with samples positive for antibodies against Dengue virus (5,0% vs. 62,5%), Zika virus (17,9% vs. 97,4%) and West Nil virus (0,0% vs. 29,4 %) (Fig. 4).

### Conclusion

Based on the data shown above, GpE was selected for the anti-POWV IgM ELISA. This antigen allowed development of a specific test which shows highly sensitive detection of positive samples while the level of cross reactivity remains low.

NS1 was selected for the anti-POWV IgG ELISA. This test is supposed to be highly specific.

## Claims

1. A recombinant or chemically-synthesized polypeptide comprising SEQ ID NO1 or a variant thereof, preferably in an isolated form.

2. A diagnostically useful carrier comprising a means for specifically capturing an antibody to SEQ ID NO1, and preferably a means for specifically capturing an antibody to SEQ ID NO2, in a sample from a subject.

3. The carrier according to claim 2, wherein the diagnostically useful carrier is selected from the group comprising a bead, preferably a paramagnetic bead, a test strip, a microtiter plate, a membrane, preferably from the group comprising western blot, line blot and dot blot, a lateral flow device, a glass surface, a slide, a microarray, and a biochip.

4. A kit comprising the polypeptide according to claim 1 or the diagnostically useful carrier according to any of claims 2 to 3, preferably further comprising a means for specifically detecting a captured antibody to SEQ ID NO1 and/or a means for specifically detecting a captured antibody to SEQ ID NO2.

5. The kit according to claim 4, wherein the means for specifically detecting a captured antibody to SEQ ID NO1 is a secondary antibody recognizing IgM class antibodies.

6. The kit according to claim 5, wherein the means for specifically detecting a captured antibody to SEQ ID NO2 is a secondary antibody recognizing IgG or IgM, preferably IgG class antibodies.

7. The kit according to any of claims 4 to 6, wherein the kit further comprises a recombinant or monoclonal antibody to SEQ ID NO2 and/or a recombinant or monoclonal antibody to SEQ ID NO1.

8. A method for diagnosing, prognosing or monitoring the treatment of a virus, preferably Flavivirus, more preferably POWV infection comprising the step detecting in a sample from a subject the presence or absence of an antibody to SEQ ID NO1 and/or an antibody to SEQ ID NO2, preferably SEQ ID NO1.

9. A method for distinguishing a POWV infection from another viral infection, preferably with a Flavivirus other than POWV, comprising the step detecting in a sample from a subject the presence or absence of an antibody to SEQ ID NO1 and/or an antibody to SEQ ID NO2, preferably SEQ ID NO1.

10. A method comprising the step immobilizing on a diagnostically useful carrier a means for specifically capturing an antibody to SEQ ID NO1 and/or a means for specifically capturing an antibody to SEQ ID NO2, preferably a means for specifically capturing an antibody to SEQ ID NO1,
wherein the diagnostically useful carrier is preferably selected from the group comprising a bead, preferably a paramagnetic bead, a test strip, a microtiter plate, a membrane, preferably from the group comprising western blot, line blot and dot blot, a lateral flow device, a glass surface, a slide, a microarray and a biochip.

11. A use of a polypeptide comprising SEQ ID NO1 and/or a SEQ ID NO2 or a variant thereof for the diagnosis of a Flavivirus, preferably POWV infection or for the manufacture of a kit for the diagnosis of a Flavivirus, preferably POWV infection, wherein preferably a POWV infection is distinguished from an infection with a Flavivirus other than POWV.

12. The method or use according to any of claims 8 to 11, wherein the antibody to SEQ ID NO2 is an IgG or IgM, preferably IgG class antibody.

13. The method according to any of claims 8 to 11, wherein the antibody to SEQ ID NO1 is an IgM class antibody.

14. A use of an IgM antibody to SEQ ID NO1 or a means for specifically capturing an IgM class antibody to SEQ ID NO1 for increasing the diagnostic reliability, preferably specificity of an immunoassay for the diagnosis of a virus, preferably Flavivirus, more preferably POWV infection.

15. The method or use according to any of claims 8 to 13, wherein the sample is a blood or CSF sample, preferably a serum sample.


SEQUENCE LISTING

<110>  EUROIMMUN Medizinische Labordiagnostika AG

<120>  A novel assay for the diagnosis of viral infections [POWV]

<130>  18PP018EP

<160>  4

<170>  PatentIn version 3.5

<210>  1
<211>  399
<212>  PRT
<213>  Powassan virus

<400>  1

Ala Ser Met Arg Cys Thr His Leu Glu Asn Arg Asp Phe Val Thr Gly
1               5                   10                  15

Thr Gln Gly Thr Thr Arg Val Ser Leu Val Leu Glu Leu Gly Gly Cys
            20                  25                  30

Val Thr Ile Thr Ala Glu Gly Lys Pro Ser Ile Asp Val Trp Leu Glu
        35                  40                  45

Asp Ile Phe Gln Glu Ser Pro Ala Glu Thr Arg Glu Tyr Cys Leu His
    50                  55                  60

Ala Lys Leu Thr Asn Thr Lys Val Glu Ala Arg Cys Pro Thr Thr Gly
65                  70                  75                  80

Pro Ala Thr Leu Pro Glu Glu His Gln Ala Asn Met Val Cys Lys Arg
                85                  90                  95

Asp Gln Ser Asp Arg Gly Trp Gly Asn His Cys Gly Phe Phe Gly Lys
            100                 105                 110

Gly Ser Ile Val Ala Cys Ala Lys Phe Glu Cys Glu Glu Ala Lys Lys
        115                 120                 125

Ala Val Gly His Val Tyr Asp Ser Thr Lys Ile Thr Tyr Val Val Lys
    130                 135                 140

Val Glu Pro His Thr Gly Asp Tyr Leu Ala Ala Asn Glu Thr Asn Ser
145                 150                 155                 160

Asn Arg Lys Ser Ala Gln Phe Thr Val Ala Ser Glu Lys Val Ile Leu
                165                 170                 175

Arg Leu Gly Asp Tyr Gly Asp Val Ser Leu Thr Cys Lys Val Ala Ser
            180                 185                 190

Gly Ile Asp Val Ala Gln Thr Val Val Met Ser Leu Asp Ser Ser Lys
        195                 200                 205

Asp His Leu Pro Ser Ala Trp Gln Val His Arg Asp Trp Phe Glu Asp
    210                 215                 220

Leu Ala Leu Pro Trp Lys His Lys Asp Asn Gln Asp Trp Asn Ser Val
225                 230                 235                 240

Glu Lys Leu Val Glu Phe Gly Pro Pro His Ala Val Lys Met Asp Val
                245                 250                 255

Phe Asn Leu Gly Asp Gln Thr Ala Val Leu Leu Lys Ser Leu Ala Gly
            260                 265                 270

Val Pro Leu Ala Ser Val Glu Gly Gln Lys Tyr His Leu Lys Ser Gly
        275                 280                 285

His Val Thr Cys Asp Val Gly Leu Glu Lys Leu Lys Leu Lys Gly Thr
    290                 295                 300

Thr Tyr Ser Met Cys Asp Lys Ala Lys Phe Lys Trp Lys Arg Val Pro
305                 310                 315                 320

Val Asp Ser Gly His Asp Thr Val Val Met Glu Val Ser Tyr Thr Gly
                325                 330                 335

Ser Asp Lys Pro Cys Arg Ile Pro Val Arg Ala Val Ala His Gly Val
            340                 345                 350

Pro Ala Val Asn Val Ala Met Leu Ile Thr Pro Asn Pro Thr Ile Glu
        355                 360                 365

Thr Asn Gly Gly Gly Phe Ile Glu Met Gln Leu Pro Pro Gly Asp Asn
    370                 375                 380

Ile Ile Tyr Val Gly Asp Leu Ser Gln Gln Trp Phe Gln Lys Gly
385                 390                 395

<210>  2
<211>  454
<212>  PRT
<213>  Powassan virus

<400>  2

Gly His Met Asp Tyr Gly Cys Ala Ile Asp Pro Glu Arg Met Glu Ile
1               5                   10                  15

Arg Cys Gly Glu Gly Leu Val Val Trp Lys Glu Val Ser Glu Trp Tyr
            20                  25                  30

Asp Gly Tyr Ala Tyr His Pro Glu Ser Pro Asp Thr Leu Ala Gln Ala
        35                  40                  45

Leu Arg Glu Ala Phe Glu Arg Gly Val Cys Gly Val Val Pro Gln Asn
    50                  55                  60

Arg Leu Glu Met Ala Met Trp Arg Ser Thr Ala Pro Glu Leu Asn Leu
65                  70                  75                  80

Val Leu Ser Glu Gly Glu Ala Asn Leu Thr Ile Val Val Asp Lys Thr
                85                  90                  95

Asp Pro Ala Asp Tyr Arg Gly Gly Thr Pro Met Val Leu Lys Lys Thr
            100                 105                 110

Gly Lys Glu Ser Lys Val Ser Trp Lys Ser Trp Gly Lys Ser Ile Leu
        115                 120                 125

Trp Ser Val Pro Asp Ser Pro Arg Arg Met Met Met Gly Val Asp Gly
    130                 135                 140

Val Gly Glu Cys Pro Leu Tyr Arg Arg Ala Thr Gly Val Phe Thr Val
145                 150                 155                 160

Ala Glu Phe Gly Val Gly Leu Arg Thr Lys Val Phe Leu Asp Leu Arg
                165                 170                 175

Gly Glu Ala Ser Lys Glu Cys Asp Thr Gly Val Met Gly Ala Ala Val
            180                 185                 190

Lys Asn Gly Lys Ala Ile His Thr Asp Gln Ser Met Trp Met Ser Ser
        195                 200                 205

Phe Arg Asn Asp Thr Gly Thr Tyr Ile His Glu Leu Ile Leu Thr Asp
    210                 215                 220

Leu Arg Asn Cys Thr Trp Pro Ala Ser His Thr Ile Asp Asn Asp Gly
225                 230                 235                 240

Val Leu Asp Ser His Leu Phe Leu Pro Val Thr Leu Ala Gly Pro Arg
                245                 250                 255

Ser Lys Tyr Asn Arg Ile Pro Gly Tyr Ser Glu Gln Val Arg Gly Pro
            260                 265                 270

Trp Asp Gln Thr Pro Leu Arg Val Val Arg Asp His Cys Pro Gly Thr
        275                 280                 285

Ser Val Arg Ile Asp Ser His Cys Asp Lys Arg Gly Ala Ser Val Arg
    290                 295                 300

Ser Thr Thr Glu Ser Gly Lys Ile Ile Pro Glu Trp Cys Cys Arg Ala
305                 310                 315                 320

Cys Glu Leu Pro Pro Val Thr Phe Arg Ser Gly Thr Asp Cys Trp Tyr
                325                 330                 335

Ala Met Glu Ile Arg Pro Val His Ser Gln Gly Gly Leu Val Arg Ser
            340                 345                 350

Met Val Val Ala Leu Glu Val Thr Trp Gly Asn Asn Glu Pro Tyr Lys
        355                 360                 365

Tyr Trp Pro Gln Leu Ser Thr Asn Gly Thr Ala His Gly His Pro His
    370                 375                 380

Glu Ile Ile Leu Tyr Tyr Tyr Glu Leu Tyr Pro Thr Met Thr Val Val
385                 390                 395                 400

Val Val Ser Val Ala Thr Phe Ile Leu Leu Ser Met Val Gly Met Ala
                405                 410                 415

Ala Gly Met Cys Met Cys Ala Arg Arg Arg Cys Ile Thr Pro Tyr Glu
            420                 425                 430

Leu Thr Pro Gly Ala Thr Val Pro Phe Leu Leu Ser Leu Ile Cys Cys
        435                 440                 445

Ile Arg Thr Ala Lys Ala
    450

<210> 3
<211> 409
<212> PRT
<213> Artificial Sequence

<220>
<223> His-tagged Glycoprotein E

<400> 3

Ala Ser Met Arg Cys Thr His Leu Glu Asn Arg Asp Phe Val Thr Gly
1               5                   10                  15

Thr Gln Gly Thr Thr Arg Val Ser Leu Val Leu Glu Leu Gly Gly Cys
            20                  25                  30

Val Thr Ile Thr Ala Glu Gly Lys Pro Ser Ile Asp Val Trp Leu Glu
        35                  40                  45

Asp Ile Phe Gln Glu Ser Pro Ala Glu Thr Arg Glu Tyr Cys Leu His
    50                  55                  60

Ala Lys Leu Thr Asn Thr Lys Val Glu Ala Arg Cys Pro Thr Thr Gly
65                  70                  75                  80

Pro Ala Thr Leu Pro Glu Glu His Gln Ala Asn Met Val Cys Lys Arg
                85                  90                  95

Asp Gln Ser Asp Arg Gly Trp Gly Asn His Cys Gly Phe Phe Gly Lys
            100                 105                 110

Gly Ser Ile Val Ala Cys Ala Lys Phe Glu Cys Glu Glu Ala Lys Lys
        115                 120                 125

Ala Val Gly His Val Tyr Asp Ser Thr Lys Ile Thr Tyr Val Val Lys
    130                 135                 140

Val Glu Pro His Thr Gly Asp Tyr Leu Ala Ala Asn Glu Thr Asn Ser
145                 150                 155                 160

Asn Arg Lys Ser Ala Gln Phe Thr Val Ala Ser Glu Lys Val Ile Leu
                165                 170                 175

Arg Leu Gly Asp Tyr Gly Asp Val Ser Leu Thr Cys Lys Val Ala Ser
            180                 185                 190

Gly Ile Asp Val Ala Gln Thr Val Val Met Ser Leu Asp Ser Ser Lys
        195                 200                 205

Asp His Leu Pro Ser Ala Trp Gln Val His Arg Asp Trp Phe Glu Asp
    210                 215                 220

Leu Ala Leu Pro Trp Lys His Lys Asp Asn Gln Asp Trp Asn Ser Val
225                 230                 235                 240

Glu Lys Leu Val Glu Phe Gly Pro Pro His Ala Val Lys Met Asp Val
                245                 250                 255

Phe Asn Leu Gly Asp Gln Thr Ala Val Leu Leu Lys Ser Leu Ala Gly
            260                 265                 270

Val Pro Leu Ala Ser Val Glu Gly Gln Lys Tyr His Leu Lys Ser Gly
        275                 280                 285

His Val Thr Cys Asp Val Gly Leu Glu Lys Leu Lys Leu Lys Gly Thr
    290                 295                 300

Thr Tyr Ser Met Cys Asp Lys Ala Lys Phe Lys Trp Lys Arg Val Pro
305                 310                 315                 320

Val Asp Ser Gly His Asp Thr Val Val Met Glu Val Ser Tyr Thr Gly
                325                 330                 335

Ser Asp Lys Pro Cys Arg Ile Pro Val Arg Ala Val Ala His Gly Val
            340                 345                 350

Pro Ala Val Asn Val Ala Met Leu Ile Thr Pro Asn Pro Thr Ile Glu
        355                 360                 365

Thr Asn Gly Gly Gly Phe Ile Glu Met Gln Leu Pro Pro Gly Asp Asn
    370                 375                 380

Ile Ile Tyr Val Gly Asp Leu Ser Gln Gln Trp Phe Gln Lys Gly Leu
385                 390                 395                 400

Glu His His His His His His His His
                405

<210>  4
<211>  464
<212>  PRT
<213>  Artificial Sequence

<220>
<223>  His-tagged NS1

<400>  4

Gly His Met Asp Tyr Gly Cys Ala Ile Asp Pro Glu Arg Met Glu Ile
1               5                   10                  15

Arg Cys Gly Glu Gly Leu Val Val Trp Lys Glu Val Ser Glu Trp Tyr
            20                  25                  30

Asp Gly Tyr Ala Tyr His Pro Glu Ser Pro Asp Thr Leu Ala Gln Ala
        35                  40                  45

Leu Arg Glu Ala Phe Glu Arg Gly Val Cys Gly Val Val Pro Gln Asn
    50                  55                  60

Arg Leu Glu Met Ala Met Trp Arg Ser Thr Ala Pro Glu Leu Asn Leu
65                  70                  75                  80

Val Leu Ser Glu Gly Glu Ala Asn Leu Thr Ile Val Val Asp Lys Thr
                85                  90                  95

Asp Pro Ala Asp Tyr Arg Gly Gly Thr Pro Met Val Leu Lys Lys Thr
            100                 105                 110

Gly Lys Glu Ser Lys Val Ser Trp Lys Ser Trp Gly Lys Ser Ile Leu
        115                 120                 125

Trp Ser Val Pro Asp Ser Pro Arg Arg Met Met Met Gly Val Asp Gly
    130                 135                 140

Val Gly Glu Cys Pro Leu Tyr Arg Arg Ala Thr Gly Val Phe Thr Val
145                 150                 155                 160

Ala Glu Phe Gly Val Gly Leu Arg Thr Lys Val Phe Leu Asp Leu Arg
                165                 170                 175

Gly Glu Ala Ser Lys Glu Cys Asp Thr Gly Val Met Gly Ala Ala Val
            180                 185                 190

Lys Asn Gly Lys Ala Ile His Thr Asp Gln Ser Met Trp Met Ser Ser
        195                 200                 205

Phe Arg Asn Asp Thr Gly Thr Tyr Ile His Glu Leu Ile Leu Thr Asp
    210                 215                 220

Leu Arg Asn Cys Thr Trp Pro Ala Ser His Thr Ile Asp Asn Asp Gly
225                 230                 235                 240

Val Leu Asp Ser His Leu Phe Leu Pro Val Thr Leu Ala Gly Pro Arg
                245                 250                 255

Ser Lys Tyr Asn Arg Ile Pro Gly Tyr Ser Glu Gln Val Arg Gly Pro
            260                 265                 270

Trp Asp Gln Thr Pro Leu Arg Val Val Arg Asp His Cys Pro Gly Thr
        275                 280                 285

Ser Val Arg Ile Asp Ser His Cys Asp Lys Arg Gly Ala Ser Val Arg
    290                 295                 300

Ser Thr Thr Glu Ser Gly Lys Ile Ile Pro Glu Trp Cys Cys Arg Ala
305                 310                 315                 320

Cys Glu Leu Pro Pro Val Thr Phe Arg Ser Gly Thr Asp Cys Trp Tyr
                325                 330                 335

Ala Met Glu Ile Arg Pro Val His Ser Gln Gly Gly Leu Val Arg Ser
            340                 345                 350

Met Val Val Ala Leu Glu Val Thr Trp Gly Asn Asn Glu Pro Tyr Lys
        355                 360                 365

Tyr Trp Pro Gln Leu Ser Thr Asn Gly Thr Ala His Gly His Pro His
    370                 375                 380

Glu Ile Ile Leu Tyr Tyr Tyr Glu Leu Tyr Pro Thr Met Thr Val Val
385                 390                 395                 400

Val Val Ser Val Ala Thr Phe Ile Leu Leu Ser Met Val Gly Met Ala
                405                 410                 415

Ala Gly Met Cys Met Cys Ala Arg Arg Arg Cys Ile Thr Pro Tyr Glu
            420                 425                 430

Leu Thr Pro Gly Ala Thr Val Pro Phe Leu Leu Ser Leu Ile Cys Cys
        435                 440                 445

Ile Arg Thr Ala Lys Ala Leu Glu His His His His His His His His
    450                 455                 460